# EUROPEAN PATENT SPECIFICATION

(11) **EP 3 969 909 B1**
(45) Date of publication and mention of the grant of the patent: **09.04.2025**
(21) Application number: 20742810.3
(22) Date of filing: 13.05.2020
(51) Int. Cl.: G01N 33/543, G05D 23/24

(54) **A SENSOR DEVICE**
SENSORVORRICHTUNG
DISPOSITIF CAPTEUR

(30) Priority: 13.05.2019 GB 201906710
(43) Date of publication of application: 23.03.2022
(73) Proprietor: University of Newcastle-Upon Tyne, Newcastle upon Tyne NE1 7RU (GB)
(72) Inventor: PEETERS, Marloes, Manchester, Greater Manchester M15 6BH (GB)
(74) Representative: Aldridge, Christopher Simon
(86) International application number: PCT/GB2020/051168
(87) International publication number: WO 2020/229822

(56) References cited:
- US-A1- 2015 160 242
- "Handbook of Molecular Imprinting - Advanced Sensor Applications", 18 September 2012, PAN STANFORD PUBLISHING PTE. LTD., Singapore, ISBN: 978-981-4316-65-1, article R RAJKUMAR ET AL: "MIP thermistor", pages: 181 - 216, XP055742087
- KRISTIAN LETTAU ET AL: "A Bifunctional Molecularly Imprinted Polymer (MIP): Analysis of Binding and Catalysis by a Thermistor", ANGEWANDTE CHEMIE, vol. 45, no. 42, 27 October 2006 (2006-10-27), DE, pages 6986 - 6990, XP055742338, ISSN: 1433-7851, DOI: 10.1002/anie.200601796
- FRANCESCO CANFAROTTA ET AL: "A novel thermal detection method based on molecularly imprinted nanoparticles as recognition elements", NANOSCALE, vol. 10, no. 4, 1 January 2018 (2018-01-01), United Kingdom, pages 2081 - 2089, XP055742325, ISSN: 2040-3364, DOI: 10.1039/C7NR07785H
- K. BETLEM ET AL: "Thermistors coated with molecularly imprinted nanoparticles for the electrical detection of peptides and proteins", ANALYST, vol. 145, no. 16, 1 January 2020 (2020-01-01), UK, pages 5419 - 5424, XP055742315, ISSN: 0003-2654, DOI: 10.1039/D0AN01046D

## Description

### Field of the Invention

The invention relates to a sensor and a sensing device, including a sensor and sensing device for detecting predetermined molecules and biomolecules.

### Background to the Invention

There is a desire to detect the presence of molecules in substances and this is particularly the case in respect of biomolecules, such as proteins and primary and secondary metabolites. The presence of such molecules can be indicative of the presence of bacteria or allergens in a substance, which is of particular concern in the food industry and environmental monitoring, for example, the testing of water quality. Furthermore, the detection of biomarkers is especially useful in medicine and medical diagnoses.

The detection and quantification of specific proteins in samples, such as biological, environmental or food samples, can be a complex and expensive process and biosensors and/or biomimetic sensors can, advantageously, offer protein detection solutions that are fast, simple, cheap and portable. However, existing biosensors can be large and require specific conditions in which to work. For example, EP3035055 discloses a biosensor device for detecting a predetermined target analyte. An aptamer bioreceptor for specifically binding to the predetermined target analyte is exposed at a functionalized surface of a substrate. The device also requires a heat source for heating the substrate via a back surface thereof. The device has a first temperature sensing element for sensing a temperature at the back side of the substrate and a second temperature sensing element for sensing a temperature at the functionalized side of the substrate. A signal processing unit is employed for calculating a heat transfer resistivity value based on temperature values obtained from the first and the second temperature sensing element and the heating power generated by the heat source. The heat source requires careful monitoring and retaining at a constant temperature to ensure a reliable reading from the device. This requires that the heat source and sample to be tested are sealed within a chamber, thereby making the device complicated to operate and it cannot be employed with continuously flowing samples. Furthermore, such equipment is bulky and required considerable processing power to monitor the temperature of the heat source and control the same. There are also limitations to the use of aptamers, which can be of limited stability and the selection procedure to obtain the aptamer may be complicated.

US 2015/160242 discloses a polymer matrix with target binding sites directed to the use of the separation or extraction of creatine from complex mixtures.

R Rajkumar ET AL: "MIP thermistor", and Kristian Lettau ET AL: "A Bifunctional Molecularly Imprinted Polymer (MIP): Analysis of Binding and Catalysis by a Thermistor" both disclose method of using molecularly imprinted polymers in conjunction with a thermistor to measure a substrate conversion in catalytic sites and it's adsorption at binding sites.

Francesco Canfarotta ET AL: "A novel thermal detection method based on molecularly imprinted nanoparticles as recognition elements" discloses a method of using nanoMIPs dipcoated onto thermocouples to detect target molecules.

### Summary of the Invention

The invention is disclosed in the appended claims.

The invention is directed to a thermistor comprising a functionalised nanoparticle coating bonded thereto, characterized in that the functionalised nanoparticle coating comprises a nanoscale molecularly imprinted polymer.

Using a functionalise nanoparticle coating allows the thermistor to become sensitive to a particular target molecule. When the target molecule is present, it binds to the coating, which reduces the heat flow to the thermistor. When such molecules bind to the coating of the thermistor, the thermal conductivity of the thermistor changes and so the resistance of the thermistor in a sample also changes, thereby indicating when the molecule is present in the sample. It is likely that the detected temperature will be different from the temperature of the sample, thereby changing the resistance of the thermistor in such a manner to be indicative of the presence of such target molecules. The temperature detected may be higher or lower according to the type of thermistor employed. Thus, the thermistor, having a functionalise nanoparticle coating, can be used to detect the presence of a target molecule by measuring a change of resistance or thermal conductivity of the thermistor, in response to the binding of target molecules to the functionalise nanoparticle coating.

An advantage of the use of thermistors over thermocouples is that the use of thermocouples results in a large signal to noise ratio, thereby resulting in inaccurate results. By employing a thermistor with a functionalised nanoparticle coating, the signal to noise ratio is much lower, which results in a more accurate reading that also requires less filtering and processing.

Additionally, with the use of thermistors in such an arrangement, rather than heating a substrate and employing thermocouples is that the thermistor is able to provide a more accurate indication of the presence of target molecules. This is, in part, because there is no need to heat the substrate, which introduces power fluctuations, and so there is less noise in the system, thereby allowing a more accurate determination of the resistance of the thermistor. Furthermore, the target molecules bind directly to the temperature sensor - the thermistor - rather than to a substrate adjacent which the temperature sensor is positioned. Thus, with the reduced signal to noise ratio obtained from the use of the thermistor, it is possible to increase the sensitivity of the device and for more accurate results can be obtained. Rather than monitoring voltage changes, as is the case with thermocouples, the measurement of resistance and thermistors can provide quicker readings than thermocouples, thereby making them better for providing real-time information for in-sample, in-liquid or in vivo situations.

It should also be noted that thermocouples are more susceptible to calibration drift with use over time compared with thermistors and so the use of thermocouples is more likely to result in inaccurate results.

It may be preferable to provide a nanoscale imprinted polymer that is sensitive to a plurality of different molecules, thereby allowing the detection of a plurality of molecules with a single test. Additionally, or alternatively, it may be advantageous to provide the coating in the form of a plurality of different nanoscale imprinted polymers, preferably, with those polymers each being sensitive to different molecules.

The invention extends to a sensing device comprising a resistance measuring device connected to the thermistor. The resistance measuring device monitors the resistance of the thermistor, the signal from which can be observed to identify changes in the resistance, which is indicative of the binding of molecules to the functionalise nanoparticle, or molecularly imprinted polymer coating. The resistance measuring device may be a multimeter or ohmmeter, or a more complex processing device, such as a computer. This enables the device to be handheld and can remove the need for a mains power supply. Additionally, as a heating element is not required, the power requirement of the device is relatively low. The change in temperature due to the presence of target molecules may be small and so the use of the thermistor allows for accurate detection of changes.

Preferably, the sensing device comprises a plurality of thermistors and at least one of those thermistors is a thermistor according to the present invention. In such a device, there is a plurality of thermistors present in which at least one of those thermistors is provided with the functionalised nanoparticle coating. This allows the device to obtain a control reading from a thermistor that is not provided with the functionalise nanoparticle coating and a sensor reading from the thermistor that is provided with such a coating. The two or more thermistors may be part of a probe. In such an arrangement, the device can be calibrated quickly and easily and the readings and relative difference between the control and the active thermistor can be readily obtained. Each thermistor may be connected to its own resistance measuring device or a single device may be employed to monitor all of the thermistors.

Preferably, the sensing device is a biomimetic sensor for detecting the presence of biomolecules in a sample, which allows the device to be employed for detecting bacteria and/or allergens in food or water and may be used in the detection of biomarkers in samples from humans and/or animals. For example, proteins, such as trypsin, biotin and epidermal growth factor receptor may be detected by the synthetic recognition elements in the functionalise coating. Furthermore, cardiac biomarkers may be detected.

Preferably, the functionalised nanoparticle coating is applied directly to the end of the thermistor and, advantageously, this is done by way of a dip-coating method. Thus, the molecularly imprinted polymer is attached to the end of a thermistor by immersing the sensitive end of the thermistor in a receptacle containing the molecularly imprinted polymer and allowing it to drain. The coated piece can then be dried by force-drying or baking. This allows for the direct detection in changes in electrical resistance, opposed to, for example, enthalpy changes.

The invention further extends to a method of detecting for the presence of a molecule comprising the steps of:
inserting the thermistor as set out in any preceding claim into a sample, wherein the coating of the thermistor is sensitive to the molecule to be detected;
monitoring the resistance of the functionalised nanoparticle coated thermistor to identify any change therein when inserted into the sample; and
using any detected change in the resistance of the functionalised nanoparticle coated thermistor to determine the presence of the target molecule.

The method may be employed in relation to the detection of a biomolecule. The sample may be a sample taken from a human or an animal and it may be employed for the detection of a biomolecule, including a biomarker.

### Brief Description of the Drawings

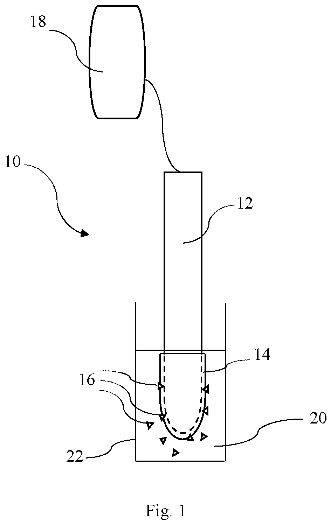

An embodiment of the invention will now be described, by way of example only, and with reference to the accompanying drawings, in which Figure 1 shows a schematic view of an arrangement according to the present invention.

### Detailed Description of Exemplary Embodiments

Figures 1 shows a biomolecule sensing device 10 comprising a thermistor 12 having a functionalised nanoparticle coating 14 bonded thereto in the form of a nanoscale molecularly imprinted polymer. The nanoscale molecularly imprinted polymer 14 is a synthetic receptor and is able to selectively bind to a specific biomolecule 16. The thermistor is connected to a resistance monitoring device 18, in the form of a multimeter or ohmmeter.

The thermistor 12 is inserted into a sample 20 within a receptacle 22 and the biomolecules 16 therein bind with the nanoscale molecularly imprinted polymer coating 14. The binding of the biomolecules to the thermistor coating insulates the thermistor and so the temperature detected by the thermistor is lower than that of the sample. This induces an electrical resistance within the thermistor that is lower than anticipated, thereby indicating that the biomolecule 16 has bound to the functionalised coating 14. Thus, the biomolecule is detected within the sample 20.

The coating may be applied to the thermistor in a number of ways.

In a first method, the thermistor is dip-coated. In this arrangement, the sensitive point of the thermistor is dipped into an aqueous solution of nanoscale molecularly imprinted polymer. The thermistor is subsequently withdrawn at a specific rate with a programmable syringe pump. Subsequently, the thermistor is washed with ethanol to remove excess particles that are not attached to the thermistor and the coated thermistor is left to dry in air. The thermistor is thus functionalised and can be used as described herein.

In another method, the thermistor is coated in the nanoscale molecularly imprinted polymer using 1-ethyl-3-(3-dimethylaminopropyl)carbodiimide (EDC) / N-hydroxysuccinimide (NHS)/ coupling. In such an arrangement, the nanoscale molecularly imprinted polymer will possess both COOH and NH2 groups, both of which can be used for coupling the polymer to the thermistor. The thermistor is incubated into an EDC/NHS solution and then exposed to the nanoscale molecularly imprinted polymer for two hours. After that period of time, the excess material is washed off with ethanol.

One arrangement of the present invention employs a negative temperature coefficient thermistor that has the advantage of fast response time and working in a wide temperature range (-40 °C to 125 °C). Such a thermistor comprises a lead wire, which is composed of nickel bifilar and insulated with polyester, that is potted into polyimide tube with a thermally conductive epoxy. Subsequently, the thermistor wires are applied to a nanoscale molecularly imprinted polymer solution in water for 60 seconds and withdrawn at a rate of 5.1 cm min⁻¹ and the arrangement air dried.

These functionalised thermistor wires can be inserted into a flow cell, that may be additively manufactured, and that flow cell may be coupled to a thermal device. The flow cell can be sealed off with a copper block that serves as a heat-sink (although other materials may be employed), in which its temperature (T₁) can be steered with a proportional-integral-derivative controller connected to a power resistor, attached to the copper. The thermistor is connected to a printed circuit board. The measured resistance from the functionalised thermistor corresponds to the temperature of the liquid (T₂) at the fixed position in the flow cell. The temperature signal can be measured every second within the flow cell to determine that changes at the interface are due to binding of the biomolecules to the MIP layer, not changes in the temperature within the flow cell. For all measurements, T₁ can be controlled, for example, at 37.00 ± 0.02 °C in order to mimic biological conditions. A sample to be tested can be placed in, or directed through, the flow cell and the resistance of the thermistor measured accordingly. It may be desirable to place a thermocouple within the flow cell to monitor the properties of the sample, such as its temperature.

Whilst the present invention may be undertaken using negative temperature coefficient or positive temperature coefficient thermistors, it is preferable to use a negative temperature coefficient thermistor to increase the resistance of the thermistor upon the decrease of detected temperature.

Preferably, the thermistor is particularly sensitive in the range between 20°C and 37°C, which is especially useful for detecting biomolecules. Compared with thermocouples, thermistors have smaller dimensions, lower cost and higher sensitivity over a specific temperature range, thereby giving significant benefits over the use of thermocouples.

It will be appreciated that some thermistors are provided with a protective polyimide coating that may have to be removed prior to the application of the functionalised coating.

The thermistor may be connected to a printed circuit board and further elements may be applied to the printed circuit board, such as a processor and/or a specialised resistance meter. The printed circuit board may be particularly advantageous where a plurality of thermistors is incorporated into a system.

Flow cells may be provided into which samples to be tested can be provided. The thermistor can be readily inserted into such flow cells to determine if the predetermined molecule is present therein.

It is envisaged that in some circumstances, the binding of molecules to the nanoscale molecularly imprinted coating may increase the thermal conductivity of the thermistor, rather than decrease. In such a situation, it may be desirable to employ a positive temperature coefficient thermistor and/or to adapt the arrangement and processing of the signal accordingly.

The present invention may be used to identify small molecules, proteins or bacteria.

The arrangement of the present invention results in a more accurate detection of molecules at a reduced cost over existing systems that employ thermocouples. Furthermore, the present invention is able to be obtain in sample, or in vivo, measurements, which can assist with providing real-time indications of the presence of molecules, particularly biomolecules, including biomarkers.

## Claims

1. A thermistor (12) comprising a functionalised nanoparticle coating (14) bonded thereto, **characterised in that** the coating (14) comprises a nanoscale molecularly imprinted polymer.

2. A sensing device (10) comprising a thermistor (12) according to claim 1 and a resistance measuring device (18) connected to the thermistor (12).

3. A sensing device according to claim 2, wherein the sensing device (10) comprises a plurality of thermistors and at least one of those thermistors is the thermistor (12) according to claim 1.

4. A sensing device (10) according to claim 2 or claim 3, wherein the sensing device (10) is a biomimetic sensor for detecting the presence of biomolecules in a sample.

5. A method of detecting for the presence of a particle (16) comprising the steps of:
inserting the thermistor (12) of any preceding claim into a sample (20);
monitoring the resistance of the functionalised nanoparticle coated thermistor (12) to identify any change therein when inserted into the sample (20); and
using any change in the resistance of the functionalised nanoparticle coated thermistor (12) to determine the presence of a target molecule (16).

6. A method according to claim 5, wherein the target molecule (16) is a biomolecule.

## Patentansprüche

1. Thermistor (12), umfassend eine funktionalisierte Nanopartikelbeschichtung (14), die daran gebunden ist,
**dadurch gekennzeichnet, dass** die Beschichtung (14) ein im Nanobereich molekular geprägtes Polymer umfasst.

2. Erfassungsvorrichtung (10), umfassend einen Thermistor (12) nach Anspruch 1 und eine Widerstandsmessvorrichtung (18), die mit dem Thermistor (12) verbunden ist.

3. Erfassungsvorrichtung nach Anspruch 2, wobei die Erfassungsvorrichtung (10) eine Vielzahl von Thermistoren umfasst und mindestens einer der Thermistoren der Thermistor (12) nach Anspruch 1 ist.

4. Erfassungsvorrichtung (10) nach Anspruch 2 oder Anspruch 3, wobei die Erfassungsvorrichtung (10) ein biomimetischer Sensor zum Nachweisen des Vorhandenseins von Biomolekülen in einer Probe ist.

5. Verfahren zum Nachweisen des Vorhandenseins eines Partikels (16), die folgenden Schritte umfassend:
Einsetzen des Thermistors (12) nach einem vorhergehenden Anspruch in eine Probe (20);
Überwachen des Widerstands des funktionalisierten, mit Nanopartikeln beschichteten Thermistors (12), um eine Änderung daran zu identifizieren, wenn er in die Probe (20) eingesetzt wird; und
Verwenden einer Änderung des Widerstands des funktionalisierten, mit Nanopartikeln beschichteten Thermistors (12), um das Vorhandensein eines Zielmoleküls (16) zu bestimmen.

6. Verfahren nach Anspruch 5, wobei das Zielmolekül (16) eine Biomolekül ist.

## Revendications

1. Thermistance (12) comprenant un revêtement nanoparticulaire fonctionnalisé (14) lié à celle-ci, **caractérisée en ce que** le revêtement (14) comprend un polymère imprimé moléculairement à l'échelle nanométrique.

2. Dispositif de détection (10) comprenant une thermistance (12) selon la revendication 1 et un dispositif de mesure de résistance (18) connecté à la thermistance (12).

3. Dispositif de détection selon la revendication 2, ledit dispositif de détection (10) comprenant une pluralité de thermistances et au moins l'une de ces thermistances étant la thermistance (12) selon la revendication 1.

4. Dispositif de détection (10) selon la revendication 2 ou 3, ledit dispositif de détection (10) étant un capteur biomimétique permettant de détecter la présence de biomolécules dans un échantillon.

5. Procédé de détection de la présence d'une particule (16), comprenant les étapes suivantes :
l'insertion de la thermistance (12) selon l'une quelconque des revendications précédentes dans un échantillon (20) ;
la surveillance de la résistance de la thermistance à revêtement nanoparticulaire fonctionnalisé (12) pour y identifier toute variation lorsqu'elle est insérée dans l'échantillon (20) ; et
l'utilisation d'une quelconque variation de la résistance de la thermistance à revêtement nanoparticulaire fonctionnalisé (12) pour déterminer la présence d'une molécule cible (16).

6. Procédé selon la revendication 5, ladite molécule cible (16) étant une biomolécule.
